(19) Europäisches Patentamt
European Patent Office
Office européen des brevets

(11) Veröffentlichungsnummer: **0 330 017**
**A2**

(12) **EUROPÄISCHE PATENTANMELDUNG**

(21) Anmeldenummer: **89102214.7**

(51) Int. Cl.[4]: **A23K 1/16 , A61K 31/425**

(22) Anmeldetag: **09.02.89**

(30) Priorität: **20.02.88 DE 3805382**

(43) Veröffentlichungstag der Anmeldung:
**30.08.89 Patentblatt 89/35**

(84) Benannte Vertragsstaaten:
**AT BE CH DE ES FR GB GR IT LI LU NL SE**

(71) Anmelder: **BOEHRINGER INGELHEIM VETMEDICA GMBH**

**D-6507 Ingelheim/Rhein(DE)**

(72) Erfinder: **Reiffen, Manfred, Dr. Dipl.-Chem.**
**Matthias-Erzberger-Strasse 40**
**D-7950 Biberach 1(DE)**
Erfinder: **Rupprecht, Eckhard, Dr. Dipl.-Biol.**
**Riedbachstrasse 15**
**D-7960 Aulendorf-Tannhausen(DE)**
Erfinder: **Quirke, John Francis, Dr.**
**Metzrothstrasse 2**
**D-6530 Bingerbrück(DE)**
Erfinder: **Sommer, Mario, Dr. Dipl.-Agraring.**
**Silvanerstrasse 4A**
**D-6501 Jungenheim(DE)**

(54) **Verwendung von Thiazolen zur Leistungssteigerung bei Tieren.**

(57) Die Erfindung betrifft die Verwendung der Thiazole der Formel

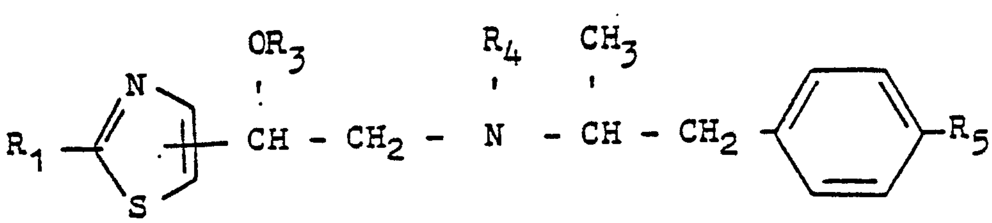

,(I)

in der
$R_1$ ein Halogenatom, eine Alkylgruppe oder eine Trifluormethylgruppe,
$R_3$ ein Wasserstoffatom oder $R_3$ und $R_4$ zusammen eine Ethylengruppe oder eine Alkoxycarbonylmethylengruppe,
$R_4$ ein Wasserstoffatom oder eine gegebenenfalls in 2- oder 3-Stellung durch eine Hydroxygruppe substituierte Alkylgruppe und
$R_5$ eine durch eine Carboxy-, Alkoxycarbonyl-, Aminocarbonyl-, Alkylaminocarbonyl-, Hydroxymethyl-, Alkoxymethyl-, Aminomethyl- oder Alkylaminomethylgruppe substituierte Methoxygruppe, eine 2-Carboxy-1-methyl-ethenyl- oder 2-Alkoxycarbonyl-1-methyl-ethenylgruppe bedeuten, deren optische Isomere und deren Diastereomere sowie deren Säureadditionssalze als leistungsfördernde Mittel bei Tieren.

EP 0 330 017 A2

## Verwendung von Thiazolen zur Leistungssteigerung bei Tieren

In der EP-A-0.239.815 werden u.a. Thiazole der Formel

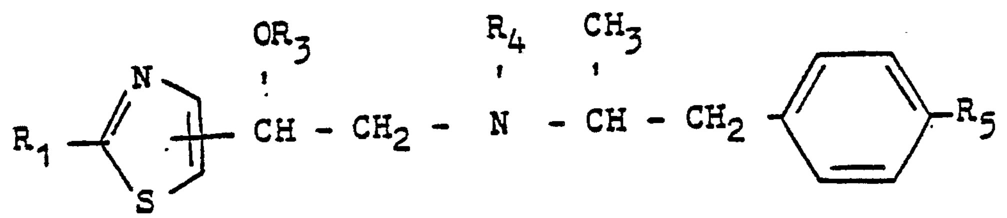 ,(I)

in der
$R_1$ ein Halogenatom, eine Alkylgruppe mit 1 bis 3 Kohlenstoffatomen oder eine Trifluormethylgruppe,
$R_3$ ein Wasserstoffatom oder $R_3$ und $R_4$ zusammen eine Ethylengruppe oder eine Alkoxycarbonylmethylengruppe, in welcher der Alkoxyteil 1 bis 3 Kohlenstoffatome enthalten kann,
$R_4$ ein Wasserstoffatom oder eine gegebenenfalls in 2- oder 3-Stellung durch eine Hydroxygruppe substituierte Alkylgruppe mit 1 bis 3 Kohlenstoffatomen und
$R_5$ eine durch eine Carboxy-, Alkoxycarbonyl-, Aminocarbonyl-, Alkylaminocarbonyl-, Hydroxymethyl-, Alkoxymethyl-, Aminomethyl- oder Alkylaminomethylgruppe substituierte Methoxygruppe, eine 2-Carboxy-1-methyl-ethenyl- oder 2-Alkoxy carbonyl-1-methyl-ethenylgruppe, wobei der Alkyl- oder Alkoxyteil jeweils 1 bis 3 Kohlenstoffatome enthalten kann, bedeuten, deren optische Isomere und deren Diastereomere sowie deren Säureadditionssalze beschrieben.

Diese Verbindungen weisen wertvolle pharmakologische Eigenschaften auf, nämlich eine Wirkung auf den Stoffwechsel, vorzugsweise eine blutzuckersenkende und körperfettreduzierende Wirkung, sowie eine senkende Wirkung auf die atherogenen ß-Lipoproteine VLDL und LDL, wobei auch einige dieser Verbindungen eine anabole Wirkung aufweisen.

Es wurde nun gefunden, daß die Thiazole der obigen Formel I auch als Leistungsförderer bei Tieren, insbesondere zur Erzielung höherer täglicher Gewichtszunahmen und verbesserter Futterausnutzung in der Tierernährung, vorzugsweise bei der Mast von Tieren wie Schafen, Schweinen, Rindern (Kälber, Ochsen, Bullen, Färsen, Kühe), Büffeln, Kaninchen und Geflügel (Tauben, Hühner, Truthühner, Perlhühner, Fasanen, Wachteln, Enten, Gänse), eingesetzt werden können.

Gegenstand der vorliegenden Erfindung ist daher die Verwendung der Thiazole der obigen Formel I, deren optische Isomere und deren Diastereomere sowie deren physiologisch verträgliche Säureadditionssalze als Leistungsförderer bei Tieren.

Physiologisch verträgliche Säureadditionssalze der Thiazole der Formel I können beispielsweise mit folgenden Säuren gebildet werden:
Salzsäure, Bromwasserstoffsäure, Schwefelsäure, Phorphorsäure, Essigesäure, Oxalsäure, Malonsäure, Fumarsäure, Maleinsäure, Bernsteinsäure, Ascorbinsäure, Äpfelsäure, Weinsäure, Milchsäure, Zitronensäure, Benzoesäure, Methansulfonsäure, Toluolsulfonsäure, Phthalsäure, Naphthalinsulfonsäure, Nikotinsäure, Palmitinsäure und Embonsäure.

Für die bei der Definition der Reste eingangs erwähnten Bedeutungen kommt beispielsweise
für $R_1$ die des Fluor-, Chlor- oder Bromatoms, der Trifluormethyl-, Methyl-, Ethyl-, n-Propyl- oder Isopropylgruppe,
für $R_3$ die des Wasserstoffatoms oder
für $R_3$ zusammen mit $R_4$ die der Ethylen-, Methoxycarbonylmethylen-, Ethoxycarbonylmethylen-, n-Propoxycarbonylmethylen- oder Isopropoxycarbonylmethylengruppe,
für $R_4$ die des Wasserstoffatoms, der Methyl-, Ethyl-, n-Propyl-, Isopropyl-, 2-Hydroxy-ethyl- oder 3-Hydroxy-n-propylgruppe und
für $R_5$ die der 2-Hydroxy-ethoxy-, 2-Methoxy-ethoxy-, 2-Ethoxy-ethoxy-, 2-n-Propoxy-ethoxy-, 2-Amino-ethoxy-, 2-Methylamino-ethoxy-, 2-Isopropylamino-ethoxy-, Carboxymethoxy-, Methoxycarbonylmethoxy-, Ethoxycarbonylmethoxy-, n-Propoxycarbonylmethoxy-, Aminocarbonylmethoxy-, Methylaminocarbonylmethoxy-, 2-Carboxy-1-methyl-ethenyl-, 2-Methoxycarbonyl1-methyl-ethenyl-, 2-Ethoxycarbonyl-1-methyl-ethenyl- oder 2-Isopropoxycarbonyl-1-methyl-ethenylgruppe in Betracht.

Bevorzugte Verbindungen der Formel I sind diejenigen, in denen

$R_1$ ein Chloratom oder eine Methylgruppe,
$R_3$ ein Wasserstoffatom oder $R_3$ und $R_4$ zusammen eine Ethylen- oder Methoxycarbonylmethylengruppe,
$R_4$ ein Wasserstoffatom, eine Methyl- oder 2-Hydroxyethylgruppe und
$R_5$ eine durch eine Carboxy-, Methoxycarbonyl-, Ethoxycarbonyl-, Aminocarbonyl-, Methylaminocarbonyl-, Hydroxymethyl- oder Ethoxymethylgruppe substituierte Methoxygruppe oder eine 2-Carbomethoxy-1-methyl-ethenylgruppe bedeuten, deren optische Isomere und deren Diastereomere sowie deren physiologisch verträglichen Säureadditionssalze.

Als besonders bevorzugte Verbindungen der Formel I seien folgende genannt:
N-[2-(4-Carbomethoxymethoxyphenyl)-1-methylethyl]-2-hydroxy-2-(2-trifluormethyl-thiazol-4-yl)ethanamin,
N-[2-(4-Aminocarbonylmethoxyphenyl)-1-methylethyl]-2-hydroxy-2-(2-trifluormethyl-thiazol-4-yl)ethanamin,
N-[2-(4-Methylaminocarbonylmethoxyphenyl)-1-methylethyl]-2-hydroxy-2-(2-trifluormethyl-thiazol-4-yl)-ethanamin,
N-[2-(4-Carbomethoxymethoxyphenyl)-1-methylethyl]-2-(2-trifluormethyl-thiazol-4-yl)morpholin,
3-[2-(4-Carbomethoxymethoxyphenyl)-1-methylethyl]-5-(2-trifluormethyl-thiazol-4-yl)-2-oxazolidincarbonsäure-methylester,
N-[2-(4-Carbomethoxymethoxyphenyl)-1-methylethyl]-N-methyl-2-hydroxy-2-(2-trifluormethyl-thiazol-4-yl)-ethanamin,
N-[2-(4-Carbomethoxymethoxyphenyl)-1-methylethyl]-N-(2-hydroxy-ethyl)-2-hydroxy-2-(2-trifluormethyl-thiazol-4-yl)ethanamin,
N-[2-(4-(2-Hydroxy-ethyl)phenyl)-1-methylethyl]-2-hydroxy-2-(2-trifluormethyl-thiazol-4-yl)morpholin,
N-[2-(4-Carboxymethoxyphenyl)-1-methylethyl]-2-(2-trifluormethyl-thiazol-4-yl)morpholin,
N-[2-(4-Carbomethoxymethoxyphenyl)-1-methylethyl]-2-hydroxy-2-(2-chlor-thiazol-4-yl)ethanamin,
N-[2-(4-(2-Ethoxy-ethyl)phenyl)-1-methylethyl]-2-hydroxy-2-(2-trifluormethyl-thiazol-4-yl)ethanamin,
N-[2-(4-(2-Hydroxy-ethoxy)phenyl)-1-methylethyl]-2-hydroxy-2-(2-trifluormethyl-thiazol-4-yl)ethanamin und
3-[2-(4-(2-Carbomethoxy-1-methylethenyl)phenyl)-1-methylethyl]-5-(2-trifluormethyl-thiazol-4-yl)-2-oxazolidin-carbonsäure-methylester

Die leistungsfördernde Wirkung der vorstehend erwähnten Thiazole wurde beispielsweise an der Verbindung N-[2-(4-Carboxymethoxyphenyl)-1-methylethenyl]-2-(2-trifluormethyl-thiazol-4-yl)morpholin wie folgt geprüft:

1.) 2 Gruppen von 7 Lämmern, wobei jedes Tier einzeln gehalten wurde, erhielten identisches Futter und Wasser ad libitum. Die eine Gruppe erhielt zusätzlich 8 ppm der vorstehend erwähnten Verbindung. Während der 33-tägigen Versuchsperiode wurden Gewichtszunahme und Futterverbrauch gegenüber der Kontrollgruppe bestimmt.

Die nachfolgende Tabelle enthält die gefundenen Ergebnisse:

| | Konzentration der Testverbindung in ppm | | Abweichung zur Kontrolle in % |
|---|---|---|---|
| | 0 | 8 | |
| Anfangsgewicht in kg | 35,6 | 35,6 | |
| Endgewicht in kg | 41,0 | 43,9 | |
| Gewichtszunahme in g/Tag | 164,0 | 258,0 | +57,3 |
| Futteraufnahme in g/Tag | 1530,0 | 1598,0 | +4,4 |
| Futterverwertung (kg Zunahme/kg Futter) | 0,107 | 0,161 | +50,5 |

2.) 2 Gruppen von 12 Lämmern erhielten identisches Futter und Wasser ad libitum. Die eine Gruppe erhielt zusätzlich 8 ppm der vorstehend erwähnten Verbindung. Während der 42-tägigen Versuchsperiode wurden Gewichtszunahme und Parameter der Schlachtkörperqualität gegenüber der Kontrolle bestimmt.

Die nachfolgende Tabelle enthält die gefundenen Ergebnisse:

| | Konzentration der Testverbindung in ppm | | Abweichung zur Kontrolle in % |
|---|---|---|---|
| | 0 | 8 | |
| Anfangsgewicht in kg | 34,8 | 33,2 | - |
| Endgewicht in kg | 47,7 | 46,6 | - |
| Gewichtszunahme g/Tag | 306,0 | 320,0 | + 4,6 |
| Rückenfettdicke (mm) | 1,87 | 1,78 | - 4,8 |
| Schlachtkörperzusammensetzung: | | | |
| - Fett (%) | 17,3 | 16,1 | -6,9 |
| - Fleisch (%) | 68,3 | 69,1 | +1,2 |
| - Knochen (%) | 14,4 | 14,8 | +2,8 |
| Gewicht des musculus longissimus dorsi in g | 303,0 | 324,0 | +6,9 |

3.) 2 Gruppen von 10 Schweinen, wobei jedes Tier einzeln gefüttert wurde, erhielten identisches Futter und Wasser ad libitum. Die eine Gruppe erhielt zusätzlich 8 ppm der vorstehend erwähnten Verbindung. Während der 33-tägigen Versuchsperiode wurden Gewichtszunahme, Futterverbrauch der Schlachtkörperqualität gegenüber der Kontrollgruppe bestimmt.

Die nachfolgende Tabelle enthält die gefundenen Ergebnisse:

| | Konzentration der Testverbindung in ppm | | Abweichung zur Kontrolle in % |
|---|---|---|---|
| | 0 | 8 | |
| Anfangsgewicht in kg | 64,6 | 65,6 | - |
| Endgewicht in kg | 96,5 | 97,6 | - |
| Gewichtszunahme g/Tag | 749,0 | 754,0 | + 0,7 |
| Futterverwertung (kg Futter/kg Zunahme) | 3,18 | 3,17 | - 0,3 |
| Ausschlachtung in % | 76,3 | 77,2 | + 1,2 |
| Gewichte verschiedener Teilstücke in kg | | | |
| - Schulter | 9,27 | 9,48 | + 2,3 |
| - Rippe | 7,11 | 7,25 | + 2,0 |
| - Schinken | 7,16 | 7,46 | + 4,2 |
| Rückenmuskelfläche $cm^2$ | 31,9 | 32,3 | + 1,3 |

Auf Grund dieser Eigenschaften können die vorstehend erwähnten Wirkstoffe als Leistungsförderer bei Tieren zur Förderung und Beschleunigung des Wachstums, der Milch- und Wollproduktion, sowie zur Verbesserung der Futterverwertung, der Schlachtkörperqualität und zur Verschiebung des Fleisch-Fett-Verhältnisses zugunsten von Fleisch eingesetzt werden. Die Wirkstoffe werden bei Nutz-, Zucht-, Zier- und Hobbytieren verwendet.

Zu den Nutz- und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Truthühner, Fische, wie z.B. Karpfen, Forellen, Lachse, Aale, Schleien, Hechte, Reptilien wie z.B. Schlangen und Krokodile.

Zu den Zier- und Hobbytieren zählen Säugetiere wie Hunde und Katzen Vögel wie Papageien Kanarienvögel Fische wie Zier-und Aquarienfische, z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichtung des gewüschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Tierart, dem

Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Tierart, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulie rungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm - 100 %, bevorzugt von 0,01 ppm - 10 %.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Futter beträgt normalerweise etwa 0,01 - 500 ppm, bevorzugt 0,1 - 50 ppm.

Die Wirkstoffe können als solche oder in Form von Prämixen oder Futterkonzentraten dem Futter zugesetzt werden.

So enthalten die erfindungsgemäßen Futtermittel neben dem Wirkstoff und gegebenenfalls neben einer üblichen Vitamin-Mineral-Mischung beispielsweise für Mastschweine Gerste, Weizennachmehl, Ackerbohnen, Raps-Extraktionsschrot und Futterfett, für Broiler Mais, Sojabohnenmehl, Fleischmehl, Futterfett und Sojaöl, für Rinder Zuckerrübenschnitzel Maiskleber, Malzkeime, Sojabohnenmehl, Weizen und Molasse sowie für Lämmer Gerste, Sojabohnenmehl, Mais und Melasse. Zu diesem Futter wird eine der eingangs erwähnten Verbindungen der Formel I als Wirkstoff, vorzugsweise jedoch N-[2-(4-Carboxymethoxyphenyl)-1-methylethenyl]-2-(2-trifluormethyl-thiazol-4-yl)morpholin, in einer Konzentration von 0,01 bis 500 ppm, vorzugsweise jedoch von 0,1 bis 50 ppm, zugemischt, wobei die Zumischung vorzugsweise in Form einer Wirkstoffvormischung. erfolgt. Diese Vormischung enthält beispielsweise pro 10 g 10 mg Wirkstoff zweckmäßigerweise in 9,99 g Maisstärke.

Die nachfolgenden Beispiele sollen die Erfindung näher erläutern:

Beispiel 1

| Alleinfutter II für Mastschweine | | |
|---|---|---|
| Gerste | 370 | g/kg |
| Weizennachmehl | 200 | g/kg |
| Maniokmehl | 135 | g/kg |
| Ackerbohnen | 100 | g/kg |
| Raps-Extraktionsschrot | 100 | g/kg |
| Futterfett lysinreiches Mineralfutter | 65 | g/kg |
| für Schweine | 20 | g/kg |
| Wirkstoff-Vormischung | 10 | g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel 2

| Mastfutter II für Broiler | | |
|---|---|---|
| Mais | 625 | g/kg |
| Sojabohnenmehl | 260 | g/kg |
| Fleischmehl | 40 | g/kg |
| Futterfett | 25 | g/kg |
| Sojaöl | 17 | g/kg |
| Bicalciumphosphat | 12 | g/kg |
| Calciumcarbonat | 6 | g/kg |
| Vitamin-/Mineralstoffmischung | 5 | g/kg |
| Wirkstoff-Vormischung | 10 | g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel 3

| Kraftfutter für Rinder | | |
|---|---|---|
| Zuckerrübenschnitzel | 600,0 | g/kg |
| Maiskleber | 100,0 | g/kg |
| Malzkeime | 50,0 | g/kg |
| Sojabohnenmehl | 35,0 | g/kg |
| Weizen | 110,0 | g/kg |
| Melasse | 60,0 | g/kg |
| Futterphosphate | 12,0 | g/kg |
| Calciumcarbonat | 2,5 | g/kg |
| Salz | 5,0 | g/kg |
| Mineralstoffe | 10,0 | g/kg |
| Vitamin-Vormischung | 5,5 | g/kg |
| Wirkstoff-Vormischung | 10,0 | g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

Beispiel 4

| Mastfutter für Lämmer | | |
|---|---|---|
| Gerste | 690 | g/kg |
| Sojabohnenmehl | 100 | g/kg |
| Mais | 150 | g/kg |
| Melasse | 30 | g/kg |
| Vitamin-/Mineralstoffmischung | 20 | g/kg |
| Wirkstoffvormischung | 10 | g/kg |

Diese Komponenten in den angegebenen Mengen ergeben nach sorgfältigem Mischen 1 kg Futter. Die 10 g Wirkstoffvormischung enthalten z.B. 10 mg Wirkstoff und 9,99 g Maisstärke.

## Ansprüche

1. Verwendung der Thiazole der Formel

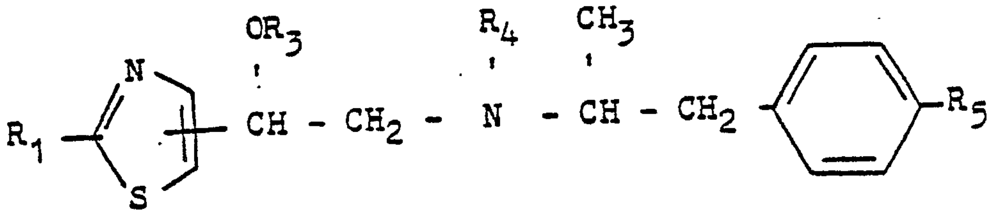

,(I)

in der

$R_1$ ein Halogenatom, eine Alkylgruppe mit 1 bis 3 Kohlenstoffatomen oder eine Trifluormethylgruppe,

$R_3$ ein Wasserstoffatom oder $R_3$ und $R_4$ zusammen eine Ethylengruppe oder eine Alkoxycarbonylmethylengruppe, in welcher der Alkoxyteil 1 bis 3 Kohlenstoffatome enthalten kann,

$R_4$ ein Wasserstoffatom oder eine gegebenenfalls in 2- oder 3-Stellung durch eine Hydroxygruppe substituierte Alkylgruppe mit 1 bis 3 Kohlenstoffatomen und

$R_5$ eine durch eine Carboxy-, Alkoxycarbonyl-, Aminocarbonyl-, Alkylaminocarbonyl-, Hydroxymethyl-, Alkoxymethyl-, Aminomethyl- oder Alkylaminomethylgruppe substituierte Methoxygruppe, eine 2-Carboxy-1-methyl-ethenyl- oder 2-Alkoxycarbonyl-1-methyl-ethenylgruppe, wobei der Alkyl- oder Alkoxyteil jeweils 1 bis 3 Kohlenstoffatome enthalten kann, bedeuten, deren optische Isomere und deren Diastereomere sowie deren physiologisch verträgliche Säureadditionssalze als leistungsfördernde Mittel bei Tieren.

2. Verwendung der Thiazole gemäß Anspruch 1, dadurch gekennzeichnet, daß ein Thiazol der Formel I, in der

$R_1$ ein Chloratom oder eine Trifluormethylgruppe,

$R_3$ ein Wasserstoffatom oder $R_3$ und $R_4$ zusammen eine Ethylen- oder Methoxycarbonylmethylengruppe,

$R_4$ ein Wasserstoffatom, eine Methyl- oder 2-Hydroxyethylgruppe und

$R_5$ eine durch eine Carboxy-, Methoxycarbonyl-, Ethoxycarbonyl-, Aminocarbonyl-, Methylaminocarbonyl-, Hydroxymethyl- oder Ethoxymethylgruppe substituierte Methoxygruppe oder eine 2-Carbomethoxy-1-methyl-ethenylgruppe bedeuten, deren optische Isomere und deren Diastereomere sowie deren physiologisch verträgliche Säureadditionssalze eingesetzt wird.

3. Verwendung von N-[2-(4-Carboxymethoxyphenyl)-1-methylethenyl]-2-(2-trifluormethyl-thiazol-4-yl)-morpholin, dessen optische Isomere und dessen Diastereomere sowie dessen physiologisch verträgliche Säureadditionssalze als leistungsförderndes Mittel bei Tieren.

4. Tiernahrung und Prämixe zur Herstellung von Tiernahrung, enthaltend mindestens ein Thiazol gemäß einem der Ansprüche 1 bis 3.

5. Leistungsförderndes Mittel für Tiere, enthaltend mindestens ein Thiazol gemäß einem der Ansprüche 1 bis 3.

6. Verfahren zur Herstellung von leistungsfördernden Mitteln für Tiere, dadurch gekennzeichnet, daß mindestens ein Thiazol gemäß einem der Ansprüche 1 bis 3 mit Streck-, Verdünnungs-und Nahrungsmitteln sowie gegebenenfalls weiteren Hilfsstoffen vermischt wird.